# EUROPÄISCHE PATENTSCHRIFT

(11) **EP 2 349 425 B1**
(45) Veröffentlichungstag und Bekanntmachung des Hinweises auf die Patenterteilung: **01.05.2013**
(21) Anmeldenummer: 09759638.1
(22) Anmeldetag: 24.11.2009
(51) Int. Cl.: A61M 16/04

(54) **SUPRAGLOTTISCHER TUBUS ZUM EINFÜHREN EINER LARYNXMASKE**
SUPRAGLOTTIC TUBE FOR INSERTING A LARYNX MASK
TUBE SUPRAGLOTTIQUE POUR INSÉRER UN MASQUE LARYNGÉ

(30) Priorität: 27.11.2008 CH 18582008; 18.11.2009 CH 17732009
(43) Veröffentlichungstag der Anmeldung: 03.08.2011
(73) Patentinhaber: Deltona Innovations AG, 8124 Maur (CH)
(72) Erfinder: DUBACH, Werner, F., CH-8124 Maur (CH)
(74) Vertreter: Schneider Feldmann AG Patent- und Markenanwälte
(86) Internationale Anmeldenummer: PCT/CH2009/000374
(87) Internationale Veröffentlichungsnummer: WO 2010/060227

(56) Entgegenhaltungen:
- WO-A1-95/16485
- US-A- 4 329 983
- US-A- 4 529 400
- US-A1- 2003 037 790
- US-A1- 2006 032 505
- US-A1- 2007 017 527
- US-A1- 2007 028 923
- US-A1- 2007 260 223
- US-A1- 2008 200 761
- US-B1- 6 210 337
- US-B1- 6 533 783

## Beschreibung

Die vorliegende Erfindung betrifft einen supraglottischen Tubus zum Einführen einer Larynxmaske über den Kehlkopf, wobei der supraglottische Tubus mehrere Lumen aufweist, wovon ein Respirationslumen der Beatmungsluftzufuhr und der Instrumentierung dient, wobei mindestens ein weiteres Lumen als Führungslumen vorhanden ist, in dem ein Führungsmittel zur Veränderung des Krümmungsradius des Tubus lagert.

Supraglottische Tuben sind Röhren, welche zur Offenhaltung der Atemwege und zur Beatmung eines Patienten in den Rachenraum eingeführt werden. Ein klassisches Beispiel sind die sogenannten Larynxmasken. Mittels des Tubus wird eine Kehlkopfmaske durch den mittleren Rachenraum über den Kehlkopfdeckel in den unteren Rachenraum eingeführt und hinter bzw. um den Kehlkopf platziert. Solche Larynxmasken dienen der Beatmung eines Patienten, während dieser anästhetisiert ist. Sie erlauben auch das Einführen von Tuben, Sonden, optischen Instrumenten und anderen Instrumenten in die Atemwege. Gleichzeitig können Larynxmasken über einen oesophagealen Zugang verfügen. Dieser erlaubt das Einführen von Sonden in die Speiseröhre und in den Magen, um Magensaft und andere Flüssigkeiten sowie Luft aus dem Magen abzusaugen. Das Entleeren des Magens bei anästhesierten Patienten soll verhindern, dass Mageninhalt in die oberen Atemwege zurückfliesst und in die ungeschützten Atemwege (Luftröhre, Bronchien und Lungen) aspiriert wird. Ein weiterer Vorteil eines oesophagealen Zugangs ist das Entweichen von passiv oder aktiv regurgitiertem Mageninhalt aus dem oberen Speiseröhrengang nach aussen, und stellt somit einen begrenzten und damit ungenügenden Aspirationsschutz dar.

Auf dem Markt ist eine grosse Anzahl unterschiedlicher Larynxmasken bekannt. Ein typisches Beispiel zeigt die US 5 878 745. Hier ist eine Gastro-Laryngealmaske gezeigt bei dem der supraglottische Tubus ein Rohr ist, durch den mehrere Schläuche geführt werden. Diese Schläuche haben Lumen, die für die Beatmung und für einen ösophagealen Zugang dienen.

Die Kombination einer Larynxmaske mit einer Oesophagealdurchführung wird immer häufiger angeboten. Auch die US-B-7040322 zeigt eine solche Larynxmaske und der supraglottische Tubus weist hier eine Unterteilung mittels einer ventral-dorsal verlaufenden Zwischenwand auf wobei im grösseren der beiden, durch die Trennung entstehenden Lumen, ein separater Schlauch eingeführt ist, der zum Aufblasen beziehungsweise Entlüften eines proximal vor der Larynxmaske dient.

Das Einführen einer Larynxmaske lässt sich mit einem relativ starren supraglottischen Tubus an sich einfacher durchführen, wobei die Steifigkeit des supraglottischen Tubus eine Anpassung der Position der Larynxmaske an die anatomischen Gegebenheiten verhindert. Weiter können starre supraglottische Tuben bei der Einführung in den Rachenraum zu Verletzungen führen und die Positionierung der Larynxmaske im Rachenraum ist nicht immer zuverlässig.

Hoch flexible supraglottische Larynxmasken-Tuben von (ProSeal® LMA, Flexible® LMA) erlauben eine bessere Positionierung um den Kehlkopf, sind aber schwieriger und damit gelegentlich traumatisch zum Einführen und schwieriger in der Positionierung im Rachenraum.
In der letzten Dekade haben sich anatomisch gebogene supraglottische Tuben zum Einführen der Larynxmaske sehr bewährt. Diese erlauben nicht nur ein einfaches Einführen der Larynxmaske in den Rachenraum und ein gutes Positionieren, sondern zeigen auch eine bessere Dichtigkeit. Solche Larynxmasken mit starrem, gebogenem supraglottischen Tubus sind insbesondere im Notfallbereich häufig im Einsatz. Wegen der anatomisch vorgeformten Gestalt ist die Einführung auch im Notfallbereich von paramedizinischen Kräften möglich. Typische Beispiele solcher Larynxmasken werden vertrieben unter der Bezeichnung LMA Fastrach® (US 5896858) und unter der Bezeichnung LMA CTrach®, beide der Firma LMA Inc. Diese gebogenen supraglottischen starren Tuben, dürfen aber wegen ihrer Starrheit nicht über längere Zeit im Patienten belassen werden und sind damit nicht für die Routineversorgung von Patienten geeignet.
Eine ähnliche Ausführung bietet auch die Firma Ambu GmbH, wobei hier die Ausführung unter der Bezeichnung Ambu Aura 40^{™} vertrieben wird. Der Bogenwinkel des supraglottischen Tubus ist jedoch grösser und weiter und das Rohr in der Konsistenz weicher. Letzteres erlaubt es diese Larynxmaske routinemässig über längere Zeit einzusetzen.

Insbesondere wenn ein Eingriff im Hals- und Kopfbereich erfolgen muss, ist die Lagerung des Kopfes sehr unterschiedlich und entsprechend kann eine Larynxmaske mit einem vorgeformten relativ starren Tubus kaum verwendet werden. Ist die Larynxmaske am Kehlkopf korrekt platziert, ist eine Fixierung in einer solchen Lage jedoch wünschenswert.

Um die Dichtigkeit der Larynxmaske um den Kehlkopf zu erhöhen, beschreibt das "ProSeal Patent" (GB 9 821 771) einen dorsalen Cuff, welcher die Larynxmaske am hochflexiblen supraglottischen Tubus von der Rachenhinterwand wegdrückt und um den Kehlkopf anpresst.

Letztlich ist aus der US 2007/0028923 eine Larynxmaske mit einem supraglottischen Tubus bekannt geworden, bei dem in der Wandung des Respirallumens ein weiterer Lumen eingeformt ist, welches zur Durchführung einer Schnur als einfaches Zugmittel aufweist. Das Zugmittel greift am distalen Ende der Larynxmaske an, wodurch auch diese gekrümmt wird. Eine gesteuerte und aktive Rückstellung ist nicht möglich.

Es ist folglich die Aufgabe der vorliegenden Erfindung einen supraglottischen Tubus zu schaffen, der in einem relativ starren Zustand ein verbessertes einfaches, anatomisches Einführen der Larynxmaske erlaubt, nach korrekter Platzierung den supraglottischen Tubus anschliessend flexibel werden lässt und bei Bedarf die Biegung des supraglottischen Tubus mit Übertragung der Biegung auf die Larynxmaske erlaubt, um in der entsprechenden Lage eine gewünschte Andruckskraft um den Kehlkopf ausüben zu können.

Diese Aufgabe löst ein supraglottischer Tubus zum Einführen einer Larynxmaske über die Epiglottis mit den Merkmalen des Patentanspruches 1.

Weitere vorteilhafte Ausgestaltungsformen des erfindungsgemässen supraglottischen Tubus gehen aus den weiteren abhängigen Ansprüchen hervor. Deren Bedeutung und Wirkungsweise ist in der nachfolgenden Beschreibung erläutert mit Bezug auf die anliegenden Zeichnungen.

Es zeigt:
- Figur 1: einen Diametralschnitt durch den erfindungsgemässen supraglottischen Tubus mit einem im Führungslumen angeordneten Führungsmittel;
- Figur 2: einen gleichen Diametralschnitt durch einen supraglottischen Tubus mit einem dorsal verstärkten Wandbereich der den Mantel eines Baudenzuges bildet.
- Figur 3: zeigt einen Längsschnitt durch einen supraglottischen Tubus mit einer proximal aufgesetzten Larynxmaske und einer distalen Verstellvorrichtung.
- Figur 4: zeigt dieselbe Anordnung wie in Figur 3, wobei der supraglottische Tubus stärker gekrümmt ist;
- Figur 5: stellt das proximale Ende eines supraglottischen Tubus in einem Diametralschnitt dar mit einem in die Larynxmaske eingreifenden Stützelement und
- Figur 6: eine Seitenansicht des supraglottischen Tubus mit einem gleich gestalteten Stützelement.
- Figur 7: zeigt einen diametralen Längschnitt durch einen supraglottischen Tubus und der Larynxmaske mit Stützelement.

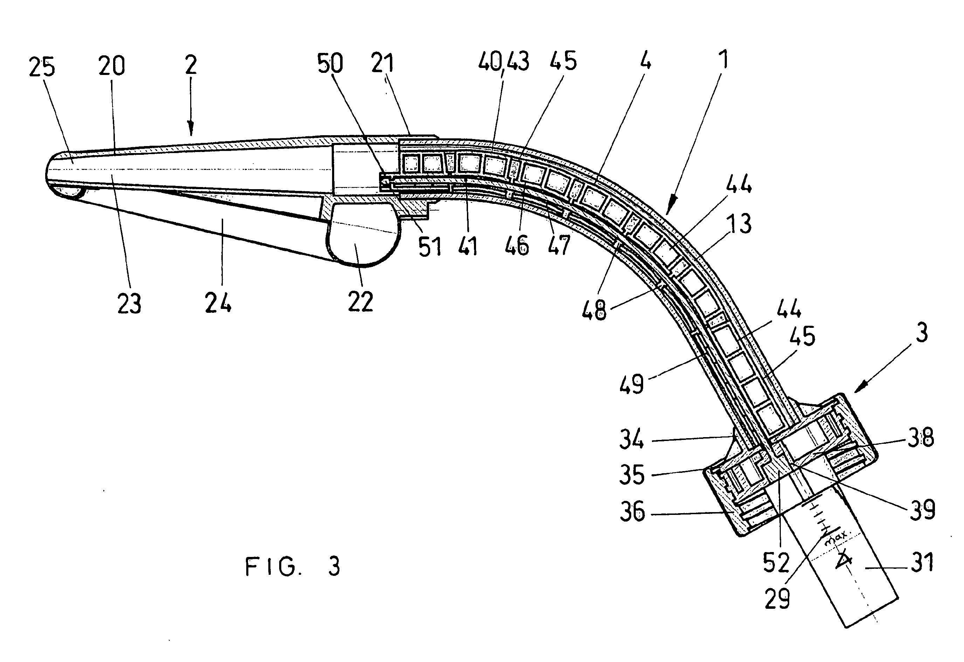

Mit Bezug auf die Figur 3 wird erst die Gesamtsituation dargestellt. Mit 1 ist der supraglottische Tubus bezeichnet. An dessen proximalen Ende ist die mit 2 bezeichnete Larynxmaske befestigt. Das distale Ende des supraglottischen Tubus ist mit einem Zufuhrstutzen und Verstelleinheit 3 versehen. Im Bezug auf die Einführungslage in den Patienten wird jene Seite mit dem kleineren, konkaven inneren Radius als die Ventralseite während jene mit dem äusseren, konvexen grösseren Radius als die Dorsalseite bezeichnet wird. Die eigentliche Larynxmaske 2, die hier in einem diametralen Längsschnitt gezeigt ist, besitzt ein Durchführungsteil 20, das in einer Aufnahmebuchse 21 an der distalen Seite endet. In dieser Aufnahmebuchse 21 findet das proximale Ende des supraglottischen Tubus 1 form- und oder kraftschlüssig Aufnahme.

Auf der unteren, ventralen Seite der Larynxmaske ist ein umlaufender Dichtkragen 22 vorhanden, der üblicherweise als Cuff 22 bezeichnet wird. Über den Cuff 22 verläuft der ösophageale Durchgang 23. Dieser Oesophagealdurchgang ist dichtend mit einem entsprechenden Lumen des supraglottischen Tubus 1 verbunden. Die Beatmungsluft strömt lateral des Oesophagealdurchgang durch und tritt in den Innenraum 24, der vom Cuff 22 dichtend um/abgeschlossen wird und von dem aus die Beatmungsluft in die Trachea einströmen beziehungsweise ausströmen kann.

Die eigentliche Ausgestaltung der Larynxmaske 2 ist für die Erfindung von sekundärer Bedeutung. Lediglich wenn am proximalen Ende des supraglottischen Tubus 1 ein Stützelement angeformt ist, so muss die Larynxmaske 2 entsprechend so ausgestaltet sein, dass dieses Stützelement darin seine korrekte Aufnahme findet. Dieses Stützelement 60 dient im Wesentlichen dazu, die Larynxmaske zu versteifen um insbesondere ein Umknicken der Spitze 25 der Larynxmaske während der Intubation zu vermeiden. Weiter hilft das Stützelement 60 die Biegung des supraglottischen Tubus auf die Kehlkopfmaske zu übertragen und ein Knicken im Bereich der Aufnahmebuchse 21 zu verhindern. Auf die exakte Ausgestaltung der Verstelleinheit und des Zufuhrstutzen 3 wird später nach der Beschreibung des supraglottischen Tubus und des Führungsmittels eingegangen.

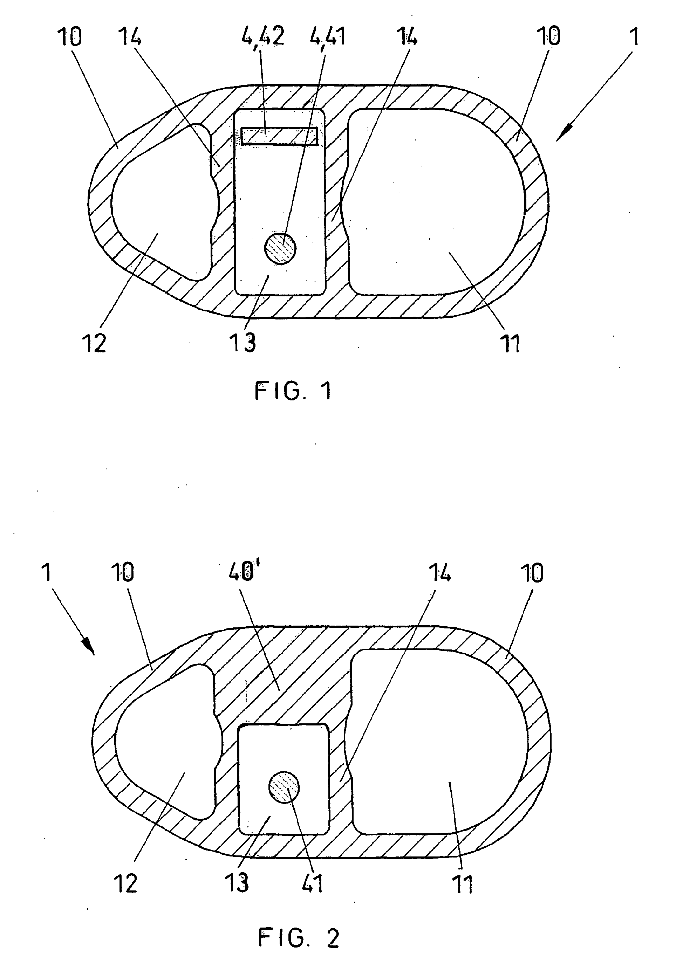

In der Figur 1 ist ein Querschnitt durch den eigentlichen supraglottischen Tubus dargestellt. Dieser besteht wie üblich aus einem hierfür zugelassenen Kunststoff. Der supraglottische Tubus 1 kann sowohl endlos extrudiert werden als auch spritzgusstechnisch hergestellt sein. Im diametralen Querschnitt gemäss der Figur 1 erkennt man deutlich die drei hier vorgesehenen Lumen. Es ist jedoch durchaus möglich, dass auch mehr als drei Lumen vorgesehen sein können, wobei man durch das eine Lumen ein eventuell erforderliches Operationsbesteck, Messsonden oder ein optisches System, beispielsweise ein Lichtleiter und / oder ein Endoskop oder beides einführen kann.

Auch hier ist wiederum der supraglottischer Tubus insgesamt mit 1 bezeichnet. Dieser besitzt im hier dargestellten Beispiel drei verschiedene Lumen. Mit 11 ist ein respiratorisches Lumen zur Durchführung der Beatmungsluft bezeichnet. Dieses Lumen besitzt üblicherweise den grössten Querschnitt. Zwischen dem Respirationslumen 11 und dem Oesophageallumen 12 liegt ein Führungslumen 13. Im Führungslumen 13 ist ein Führungsmittel 4 angeordnet. Dieses Führungsmittel 4 kann fest oder auswechselbar im Führungslumen 13 gehalten sein. Dies ist unabhängig davon, ob man den supraglottischen Tubus als Einwegteil oder als sterilisierbares wiederverwendbares Teil gestalten will. Das Führungsmittel 4 führt zu einer gewissen Versteifung des supraglottischen Tubus 1 und führt damit zu einer verbesserten Einführung der Larynxmaske in den Patienten und erlaubt andererseits eine Veränderung des Krümmungsradius des supraglottischen Tubus und somit ebenfalls eine verbesserte Einführung der Larynxmaske in den Patienten sowie eine Anpassung der Position der Larynxmaske an die Anatomie des Patienten sowie an dessen Lagerung. Entsprechend weist das Führungsmittel 4 zwei Elemente auf, nämlich ein rückfederndes und/oder veränderbares Druckorgan 40 und ein flexibles Zugorgan 41. Die Ausgestaltung dieser beiden Organe 40, 41 kann sehr unterschiedlich sein. Das Zugorgan 41 besteht immer aus einem flexiblen, und bei den hier auftretenden Kräften undehnbaren Seil, Draht oder Kabel, welches mono- oder multifilar gestaltet sein kann. Auch die dafür verwendeten Materialien können sehr unterschiedlich sein, wesentlich ist nur, dass diese für medizinaltechnische Zwecke zugelassen sind.

In einer besonders einfachen Ausgestaltungsform, wie dies in der Figur 1 dargestellt ist, kann das Druckorgan 40 ein im Querschnitt rechteckiges, relativ flaches Metallprofil sein mit einer relativ hohen Elastizität, wobei dieses Flachprofil als spezielles Ausführungsbeispiel eines Druckorgans mit 42 bezeichnet ist. Dieses Flachprofil 42 besitzt eine Krümmung, welche der Mindestkrümmung des supraglottischen Tubus 1 entspricht. Direkt oder mittelbar ist am Druckorgan 40 beziehungsweise am Flachprofil 42 am proximalen Ende das Zugorgan 41 befestigt. Im einfachsten Fall könnte das Zugorgan 41 lose am Ende des supraglottischen Tubus 1 aus diesem herausragen und der Operateur könnte durch Zug an diesem Zugorgan 41 bei gleichzeitigem Gegendruck auf den Tubus an dessen distalen Ende beziehungsweise durch Gegendruck am Zufuhrstutzen beziehungsweise der Verstelleinheit 3 die Krümmung realisieren. Zur Fixierung der Endlage liesse sich das Zugorgan 41 an einem entsprechenden Fixierungsmittel, beispielsweise einen einfachen Haken in der gewünschten Krümmung fixieren. Im hier dargestellten Beispiel jedoch ist das Zugorgan 41 fest an einem Teil des Zufuhrstutzen und Verstelleinheit 3 befestigt. Hierauf wird nachfolgend bei der Detailbeschreibung des Zufuhrstutzen und Verstelleinheit 3 noch eingegangen.

In der Figur 2 ist eine weitere Ausgestaltungsform des Führungsmittels 4 gezeigt. Während das Zugorgan 41 wiederum als einfaches Seil oder Kabel gestaltet ist, ist das Druckorgan hier mit 40' bezeichnet und ist ein integrierter Bestandteil des supraglottischen Tubus 1. Der supraglottische Tubus 1 besitzt eine relativ dünne Aussenwand 10 und die einzelnen Lumen 11, 12, 13 sind untereinander durch Trennwände 14 unterteilt.

Bei der Ausführung gemäss der Figur 2 ist die Aussenwand im Bereich des Führungslumen 13 stark verdickt. Hierdurch bildet dieser Aussenwandbereich ein Druckorgan 40'. Ein solcher spritzgusstechnisch oder stranggepresster supraglottischer Tubus kann in der Minimalkrümmung vorgeformt gefertigt sein oder kann gerade verlaufend gefertigt sein und unter Vorspannungszug oder nachträglich unter Wärmebehandlung gekrümmt werden. Das Druckorgan 40, 40' also der verdickte Wandbereich oder das Flachprofil 42 sind immer an der dorsalen Seite des supraglottischer Tubus angeordnet, während das Zugorgan auf der ventralen Seite des supraglottischer Tubus liegt. Wie bereits erwähnt führt ein entsprechender Zug ausgeübt auf das Zugorgan 41 zu einer Krümmung des supraglottischen Tubus 1; fällt dieser Zug weg, so verformt sich der supraglottische Tubus 1 einerseits durch die Eigenelastizität und oder unter Hilfe des elastischen Druckorgans 40, 40' in die ursprüngliche Form zurück.

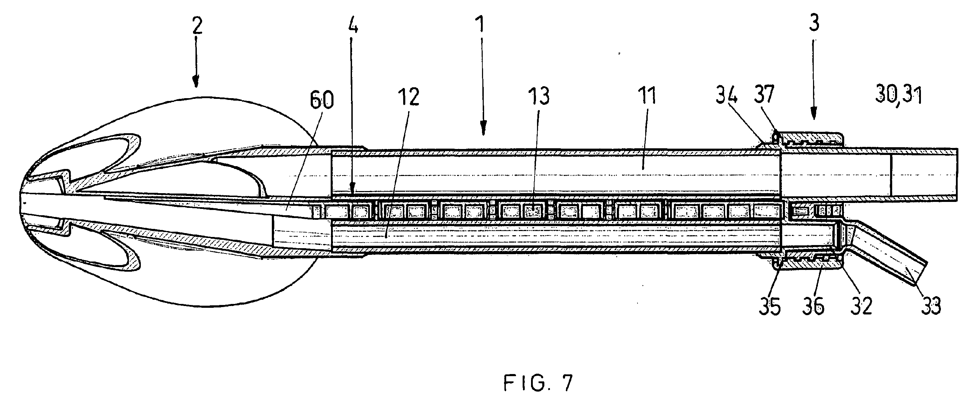

Eine besonders bevorzugte Ausführungsform des Führungsmittel 4 ist in den Figuren 3 und 7 dargestellt. Das Druckorgan 40 ist hier als eine bevorzugterweise einstückige Gliederkette 43 gestaltet. Diese Gliederkette 43 besitzt Kettenglieder 44, die unterschiedlich gross gestaltet sein können und allesamt über einer dorsalen ununterbrochenen Wand 45 zusammenhängen. Parallel zu dieser dorsalen Wand 45 verlaufen zwei parallele ventrale Wände, nämlich eine innere ventrale Wand 46 und eine äussere ventrale Wand 47. Sowohl die innere ventrale Wand 46 als auch die äussere ventrale Wand 47 weisen Unterbrüche 48 auf, die in etwa übereinanderliegend angeordnet sind. Zwischen der inneren ventralen Wand 46 und der äusseren ventralen Wand 47 verläuft ein Zwischenraum 49, in dem das Zugorgan 41 verläuft. Am proximalen Ende der Gliederkette 43 ist eine Verankerungshülse 50 angeformt. Diese Verankerungshülse 50 wird durch einen Fortsatz der inneren und äusseren ventralen Wand 46, 47 gebildet. Das Zugorgan 41 ist hier als monofilare Schnur gestaltet, an der proximal ein Kopf 51 angeformt ist. Am distalen Ende des Zugorgans 41 ist hier ein zylindrisch gestalteter Lagerkopf 52 angeformt. Die Gliederkette 43 im Führungslumen 13 dient weiter als ein Kompressions/Beisschutz für den distalen supraglottischen Tubusbereich.

Wiederum in den Figuren 3 und 7 ist der kombinierte Zufuhrstutzen und die Verstelleinheit 3 im Diametralschnitt ersichtlich. Der eigentliche Zufuhrstutzen ist mit 30 bezeichnet. Dieser Zufuhrstutzen 30, obwohl einstückig gestaltet, weist zwei verschiedene Stutzen auf, nämlich den Beatmungsluftzufuhrstutzen 31 und den Oesophagealstutzen 32. Der Beatmungsluftzufuhrstutzen 31 steht in kommunizierender Verbindung mit dem Respirationslumen 11 und der Oesophagealstutzen 32 steht in kommunizierender Verbindung mit dem Oesophageallumen 12. Der Luftzufuhrstutzen 31 verläuft gerade und fluchtend mit dem distalen Ende des Respirationslumen 11 beziehungsweise dem supraglottischen Tubus 1. Der Oesophagealstutzen 32 verläuft im direkten Anschlussbereich des Oesophageallumen 12 ebenfalls gerade und mit diesem fluchtend, besitzt aber ein abgekröpftes, aufgestecktes Ende 33. In der Figur 3 ist nur der Luftzufuhrstutzen 31 ersichtlich, da in der hier dargestellten Position der Oesophagealstutzen 32 in Sichtrichtung direkt fluchtend dahinter angeordnet ist.

Der kombinierte Zufuhrstutzen 30 besitzt eine Dichthülse 34, in den der supraglottische Tubus 1 dichtend form- und oder kraftschlüssig gehalten ist. Da in diesem Bereich weder grosse Kräfte noch hohe Drücke auftreten und das Material des supraglottische Tubus 1 elastisch ist, sollte eine rein kraftschlüssige Verbindung genügen. Es kann jedoch auch zusätzlich eine Klebeverbindung vorhanden sein oder die Dichthülse 34 kann auch zusätzlich mit ein oder mehreren umlaufenden Radialrippen versehen sein und entsprechende umlaufende Radialrillen können im supraglottischen Tubus angebracht sein um so auch zusätzlich eine formschlüssige Verbindung zu erzielen. Eine nach proximal verlängerte Dichthülse 34 über dem gerade, distalen Teil des supraglottischen Tubus 1, bietet zusätzlich einen Beisschutz. Am distalen Ende der Dichthülse 34 ist ein radial nach aussen abstehender Gleitkragen 35 angeformt. Dieser Gleitkragen 35 greift in eine umlaufenden Gleitnut 37 ein, welche im proximalen Endbereich eine Überwurfmutter 36 eingeformt ist. In der Überwurfmutter 36 läuft ein Zugzapfen 38, der am deutlichsten in der Figur 3 ersichtlich ist. Der Zugzapfen 38 verschiebt sich bei einer Drehbewegung der Überwurfmutter 36 nur in axialer Richtung relativ zum distalen Ende des supraglottischen Tubus 1. Der Zugzapfen 38 weist einen Lagerbecher 39 auf, in dem der zylindrische Lagerkopf 52 zugfest lagert. Da der Zugzapfen 38 lediglich axial bewegt wird und nicht rotiert kann der Luftzufuhrstutzen 31 den Zugzapfen 38 problemlos durchsetzen. Der Oesophagealstutzen 32 durchsetzt den Zugzapfen 38 ebenfalls, während das abgekröpfte Ende 33 am Zugzapfen 38 angeformt ist. Zwischen dem Oesophagealstutzen 32 und dem Zugzapfen 38 besteht somit eine Gleitdichtung.

Der supraglottische Tubus 1 gemäss der Erfindung lässt sich folglich aus einem relativ weichen und biegsamen Material fertigen, der sich leicht an die Anatomie anpasst. Dank dem Druckorgan ergibt sich eine relativ hohe Druckfestigkeit. Das Führungsmittel 4 erlaubt dann im eingeführten Zustand den Krümmungsradius des supraglottischen Tubus 1 lediglich durch Drehen der Überwurfmutter 36 zu verstellen. Damit versteift sich der supraglottische Tubus und lässt sich, insbesondere durch seine gebogene Form einfacher einführen und platzieren. Sekundär lässt sich der Krümmungsradius des supraglottischen Tubus an die Anatomie des Patienten beziehungsweise an dessen Lagerungsposition anpassen sodass dabei der Andruck der Larynxmaske 2 beziehungsweise dessen Cuff 22 um den Kehlkopf aufrechterhalten wird.

Ist der supraglottische Tubus 1 in den Patienten eingeführt, ist an sich kaum erkennbar, ob die Anpassung der Form beziehungsweise der Krümmungsradius des Tubus an die Anatomie des Patienten bereits erfolgt ist oder nicht. Zwar ragt bei minimalem Krümmungsradius der Beatmungsluftzufuhrstutzen 31 weiter aus dem Zugzapfen 38 heraus, doch ist dies nicht augenfällig. Daher wird vorgeschlagen, auf den Beatmungsluftzufuhrstutzen 31 eine Skala 29 (Fig. 3) anzubringen, um die relative Krümmung des Tubus ablesen zu können.

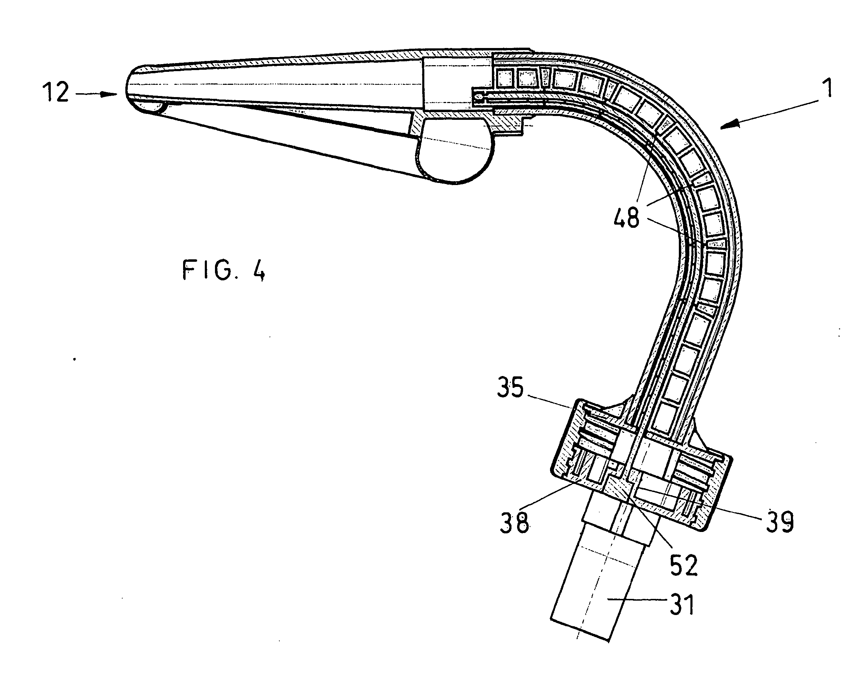

In der Figur 4 ist im Prinzip der supraglottische Tubus 1 zusammen mit der Larynxmaske 2 und dem Zufuhrstutzen 3 nochmals dargestellt, jedoch in einem stärker gekrümmten Zustand, wobei hier der Zugzapfen 38 in distaler Richtung entsprechend verschoben angeordnet ist, wodurch der Anteil des Zugorgans 41 ein grösseres Stück aus dem supraglottische Tubus 1 herausgezogen ist und somit relativ verkürzt wird, während die Länge des Druckorgans 40 unverändert bleibt.

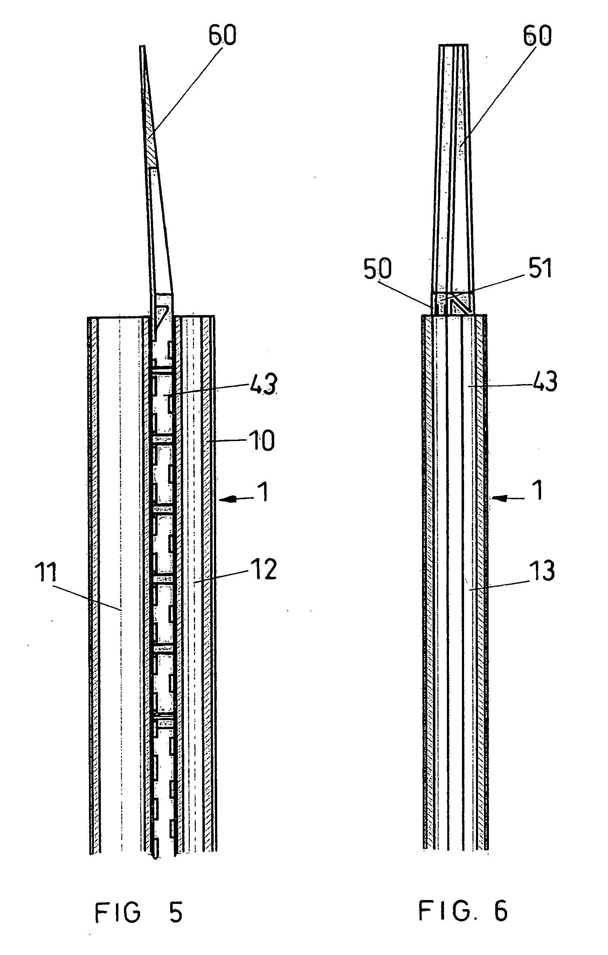

Da, wie bereits erwähnt, der Druck, der vom supraglottischen Tubus 1 aufgenommen werden kann, relativ hoch ist, ist es sinnvoll entsprechend auch Mittel vorzusehen, die zu einer Versteifung der Larynxmaske 2 dienen können, um insbesondere ein Umknicken der Larynxmaske 2 im Bereich der Aufnahmebuchse 21 beziehungsweise der Larynxmaske 2 selber und deren Spitze 25 weitgehend zu verhindern. Hierzu sind in den Figuren 5 und 6 zwei Lösungen schematisch aufgezeigt. Die Figuren 5 und 6 zeigen den supraglottischen Tubus 1 jeweils in einer Aufsicht und einer Seitenansicht, wobei hier lediglich deren proximales Ende schematisch dargestellt ist. In beiden Fällen ist hier am proximalen Ende der Gliederkette 43 jeweils ein Stützelement 60 angeformt. In der Ansicht gemäss der Figur 5 ist dieses Stützelement 60 als vertikale Stützwand ausgebildet. Diese Stützwand kann als Verlängerung des vordersten, proximalen Kettengliedes geformt sein. Dabei ist die Höhe der Stützwand 60 um die Wandstärke der Aussenwand 10 beidseitig verkürzt um eine Einführung des Stützelementes 60 in die Larynxmaske im Bereich der Aufnahmebuchse 21 problemlos zu ermöglichen. Selbstverständlich muss die Larynxmaske 2 entsprechend so gestaltet sein, dass die Stützwand 60 möglichst weit bis in den Bereich der Spitze 25 einführbar ist.

Da die Stützwand 60, wie in der Figur 5 dargestellt, praktisch senkrecht zu einer möglichen Verbiegung der Larynxmaske 2 verläuft, ist entsprechend die Stützkraft gross und daher wird diese Ausführungsform bevorzugt. Während der supraglottische Tubus selber aus einem weichen Kunststoff gefertigt ist, ist das Druckorgan 40, hier als Gliederkette 43 aus hartem Kunststoff und folglich als Stützelement 60 ausgesprochen geeignet. Das gesamte Führungsmittel 4 mit der Stützwand beziehungsweise dem Stützelement 60 lässt sich einfach in den supraglottischen Tubus 1 einführen.

Eine besonders bevorzugte Ausführungsform des Führungsmittels 4 ist, dass das Führungsmittel 4 ein fix vorgeformtes Element ist und in den supraglottischen Tubus eingebracht werden kann oder fix eingebracht ist, wobei das Element verbiegbar = anpassbar oder nicht verbiegbar sein kann.

Eine besonders bevorzugte Ausführungsform des supraglottischen Tubus enthält einsetzbar oder fix eingebaut:
a. Inflationsleitung für den Cuff durch den supraglottischen Tubus
b. Thermosensor durch den supraglottischen Tubus
c. Beatmungsdruckmessung durch den supraglottischen Tubus
d. Atemgas-Messung durch den supraglottischen Tubus
e. Optische Bildübertragung durch den supraglottischen Tubus
f. Messsonde durch den supraglottischen Tubus

Eine zusätzliche Funktion des Führungsmittel besteht darin, dass dieser als Beisschutz zu dienen vermag.

### Bezugszeichenliste:

| | |
|---|---|
| 1 | supraglottischer Tubus |
| 2 | Larynxmaske |
| 3 | Zufuhrstutzen und Verstelleinheit |
| 4 | Führungsmittel |
| 10 | Aussenwand |
| 11 | Respirationslumen |
| 12 | Oesphageallumen |
| 13 | Führungslumen |
| 14 | Trennwände |
| 20 | Durchführungsteil der Larynxmaske |
| 21 | Aufnahmebuchse |
| 22 | Cuff oder Dichtkragen |
| 23 | Oesophagealdurchgang |
| 24 | Innenraum |
| 25 | Spitze der Larynxmaske |
| 29 | Skala für Krümmungsradius |
| 30 | Zufuhrstutzen |
| 31 | Beatmungsluftzufuhrstutzen |
| 32 | Oesophagealstutzen |
| 33 | Abgekröpftes Ende von 32 |
| 34 | Dichthülse |
| 35 | Gleitkragen |
| 36 | Überwurfmutter |
| 37 | umlaufende Gleitnut |
| 38 | Zugzapfen |
| 39 | Lagerbecher |
| 40, 40' | Druckorgan |
| 41 | Zugorgan |
| 42 | Flachprofil |
| 43 | Gliederkette |
| 44 | Kettenglieder |
| 45 | dorsale Wand der Gliederkette |
| 46 | innere ventrale Wand |
| 47 | äussere ventrale Wand |
| 48 | Unterbrüche |
| 49 | Zwischenraum |
| 50 | Verankerungshülse |
| 51 | Kopf an Zugorgan 41 |
| 52 | zylindrischer Lagerkopf |
| 60 | Stützelement |

## Patentansprüche

1. Supraglottischer Tubus (1) zum Einführen einer Larynxmaske (2) über den Kehlkopf, wobei der supraglottische Tubus (1) mehrere Lumen aufweist, wovon ein Respirationslumen (11) der Beatmungsluftzufuhr und der Instrumentierung dient, wobei mindestens ein weiteres Lumen als Führungslumen (13) vorhanden ist, in dem ein Führungsmittel (4) zur Veränderung des Krümmungsradius des Tubus (1) lagert, wobei das Führungsmittel (4) ein flexibles Zugorgan (41) umfasst, **dadurch gekennzeichnet, dass** das Führungsmittel (4) zusätzlich ein Druckorgan (40) umfasst, welches eine einteilige Gliederkette (43) ist, mit einer durchgehenden dorsalen Wand (45) und mindestens einer mit Unterbrüchen (48) versehenen ventralen Wand (46, 47).

2. Supraglottischer Tubus (1) nach Anspruch 1, **dadurch gekennzeichnet, dass** an dessen distalen Ende eine Überwurfmutter (36) befestigt ist, in der ein Zugzapfen (38) relativ zum distalen Ende des supraglottischen Tubus (1) verschiebbar ist, in welcher ein Ende des Zugorgans (41) gehalten ist.

3. Supraglottischer Tubus (1) nach Anspruch 1 **dadurch gekennzeichnet, dass** zusätzlich ein oesophageales Lumen (12) vorhanden ist, welches dem oesophagealen Zugang dient.

4. Supraglottischer Tubus (1) nach Anspruch 1, **dadurch gekennzeichnet, dass** das Führungslumen (13) am proximalen Ende verschlossen ist.

5. Supraglottischer Tubus (1) nach Anspruch 3, **dadurch gekennzeichnet, dass** das Führungslumen (13) medial zwischen den beiden angrenzenden Respirations- und Oesophageallumen (11, 12) angeordnet ist.

6. Supraglottischer Tubus (1) nach Anspruch 1, **dadurch gekennzeichnet, dass** er einstückig aus einem Rohr gebildet ist und die Lumen durch ventral-dorsal verlaufende Trennwände (14) gebildet sind.

7. Supraglottischer Tubus (1) nach Anspruch 5, **dadurch gekennzeichnet, dass** mindestens eine Trennwand (14) am proximalen Ende verlängert ist und als ventral-dorsal verlaufendes Stützelement in die Larynxmaske (2) eingreift.

8. Supraglottischer Tubus (1) nach Anspruch 6, **dadurch gekennzeichnet, dass** das Stützelement (60, 60') sich bis zur proximalen mindestens annähernd in die Spitze der Larynxmaske (25) erstreckt.

9. Supraglottischer Tubus (1) nach Anspruch 1, **dadurch gekennzeichnet, dass** das flexible Zugorgan (41) am proximalen Ende des Druckorgans (40) zugfest verankert ist.

10. Supraglottischer Tubus (1) nach Anspruch 1, **dadurch gekennzeichnet, dass** zwei parallele ventrale Wände (46, 47) mit Unterbrüchen (48) vorhanden sind und zwischen diesen beiden Wänden (46, 47) das Zugorgan (41) verläuft.

11. Supraglottischer Tubus (1) nach Anspruch 1, **dadurch gekennzeichnet, dass** am proximalen Ende des Druckorgans (40, 43) ein Stützelement angeformt ist, welches mindestens bis annähernd zur Spitze (25) der Larynxmaske (2) in diese einschiebbar ist.

## Claims

1. A supraglottic tube (1) for inserting a larynx mask (2) via the larynx, wherein the supraglottic tube (1) has several lumens, of which one respiration lumen (11) serves for the supply of respiration air and for instrumentation, wherein at least one further lumen is present as a guide lumen (13), in which a guide means (4) is positioned for changing the radius of curvature of the tube (1), wherein the guide means (4) comprises a flexible traction element (41), **characterized in that** the guide means (4) comprises in addition a pressure element (40), which is a one-piece link chain (43), with a continuous dorsal wall (45) and with at least one ventral wall (46, 47) provided with interruptions (48).

2. The supraglottic tube (1) according to Claim 1, **characterized in that** at its distal end a union nut (36) is fastened, in which a traction pin (38) is displaceable relative to the distal end of the supraglottic tube (1), in which an end of the traction element (41) is held.

3. The supraglottic tube (1) according to Claim 1, **characterized in that** in addition an oesophageal lumen (12) is present, which serves for oesophageal access.

4. The supraglottic tube (1) according to Claim 1, **characterized in that** the guide lumen (13) is closed at the proximal end.

5. The supraglottic tube (1) according to Claim 3, **characterized in that** the guide lumen (13) is arranged medially between the two adjoining respiration and oesophageal lumens (11, 12).

6. The supraglottic tube (1) according to Claim 1, **characterized in that** it is formed in one piece from a tube, and the lumens are formed by separating walls (14) running ventral-dorsally.

7. The supraglottic tube (1) according to Claim 5, **characterized in that** at least one separating wall (14) is extended at the proximal end and engages into the larynx mask (2) as a supporting element running ventral-dorsally.

8. The supraglottic tube (1) according to Claim 6, **characterized in that** the supporting element (60, 60') extends up to the proximal at least approximately into the tip (25) of the larynx mask (2).

9. The supraglottic tube (1) according to Claim 1, **characterized in** the flexible traction element (41) is anchored firmly at the proximal end of the pressure element (40).

10. The supraglottic tube (1) according to Claim 1, **characterized in that** two parallel ventral walls (46, 47) with interruptions (48) are present, and the traction element (41) runs between these two walls (46, 47).

11. The supraglottic tube (1) according to Claim 1, **characterized in that** a supporting element is formed at the proximal end of the pressure element (40, 43), which supporting element is able to be inserted at least approximately to the tip (25) of the larynx mask (2) into the latter.

## Revendications

1. Tube supraglottique (1) destiné à l'introduction d'un masque laryngé (2) au-dessus du larynx, sachant que le tube supraglottique (1) présente plusieurs passages, desquels un passage de respiration (11) sert à l'amenée d'air respiratoire et à l'instrumentation, sachant qu'au moins un passage supplémentaire est présent en tant que passage de guidage (13) dans lequel un moyen de guidage (4) repose pour modifier l'angle de courbure du tube (1), sachant que le moyen de guidage (4) comprend un organe de traction flexible (41), **caractérisé en ce que** le moyen de guidage (4) comprend en outre un organe de pression (40) qui est une chaîne à maillons d'une seule pièce (43), comprenant une paroi dorsale traversante (45) et au moins une paroi ventrale (46, 47) dotée de discontinuités (48).

2. Tube supraglottique (1) selon la revendication 1, **caractérisé en ce qu'**un écrou-raccord (36) est fixé sur son extrémité distale, dans lequel un ergot de traction (38) est mobile par rapport à l'extrémité distale du tube supraglottique (1), dans lequel une extrémité de l'organe de traction (41) est maintenue.

3. Tube supraglottique (1) selon la revendication 1, **caractérisé en ce qu'**un passage oesophagien (12) est prévu en plus, lequel sert à l'accès à l'oesophage.

4. Tube supraglottique (1) selon la revendication 1, **caractérisé en ce que** le passage de guidage (13) est fermé sur l'extrémité proximale.

5. Tube supraglottique (1) selon la revendication 3, **caractérisé en ce que** le passage de guidage (13) est disposé de manière médiane entre les deux passages respiratoire et oesophagien (11, 12).

6. Tube supraglottique (1) selon la revendication 1, **caractérisé en ce qu'**il est formé d'une seule pièce à partir d'un tube et que les passages sont formés par des parois de séparation (14) au parcours ventro-dorsal.

7. Tube supraglottique (1) selon la revendication 5, **caractérisé en ce qu'**au moins une paroi de séparation (14) est prolongée sur l'extrémité proximale et se met en prise dans le masque laryngé (2) en tant qu'élément d'appui au parcours ventro-dorsal.

8. Tube supraglottique (1) selon la revendication 6, **caractérisé en ce que** l'élément d'appui (60, 60') s'étend vers la proximale, au moins approximativement dans la pointe (25) du masque laryngé (2).

9. Tube supraglottique (1) selon la revendication 1, **caractérisé en ce que** l'organe de traction flexible (41) est ancré en résistance à la traction sur l'extrémité proximale de l'organe de pression (40).

10. Tube supraglottique (1) selon la revendication 1, **caractérisé en ce que** deux parois ventrales parallèles (46, 47) avec des discontinuités (48) sont présentes et que l'organe de traction (41) passe entre ces deux parois (46, 47).

11. Tube supraglottique (1) selon la revendication 1, **caractérisé en ce qu'**un élément d'appui est formé sur l'extrémité proximale de l'organe de pression (40, 43), lequel peut être introduit dans le masque laryngé (2) au moins jusqu'à approximativement la pointe (25) de celui-ci.
